# EUROPEAN PATENT APPLICATION

(11) **EP 4 265 208 A1**
(43) Date of publication of application: **25.10.2023**
(21) Application number: 21905620.7
(22) Date of filing: 09.12.2021
(51) Int. Cl.: A61B 18/12

(54) **RADIO FREQUENCY ABLATION APPARATUS**

(30) Priority: 17.12.2020 CN 202011501204
(71) Applicant: Hangzhou Nuo Cheng Medical Instrument Co., Ltd, Hangzhou, Zhejiang 310000 (CN)
(72) Inventor: LIU, Daoyang, Hangzhou, Zhejiang 310000 (CN); WANG, Xiongzhi, Hangzhou, Zhejiang 310000 (CN); QIU, Xinjiong, Hangzhou, Zhejiang 310000 (CN); ZHANG, Tingchao, Hangzhou, Zhejiang 310000 (CN)
(74) Representative: KIPA AB
(86) International application number: PCT/CN2021/136923
(87) International publication number: WO 2022/127691

(57) **Abstract**

A radio frequency ablation apparatus (100), comprising a radio frequency energy generation unit (23), a signal acquisition unit (21), a control unit (22) and an ablation component (24), wherein the radio frequency energy generation unit (23) generates and outputs a radio frequency power required for radio frequency ablation, and transmits same to an ablation site of a heart by means of the ablation component (24); the signal acquisition unit (21) is in contact with a plurality of different body surface sites of a patient (200), so as to acquire a multi-lead ECG signal represented by a cardiac electrophysiological activity acting on the surface of the body during a radio frequency ablation process; the control unit (22) controls, according to the multi-lead ECG signal, the radio frequency power output by the radio frequency energy generation unit (23); and the radio frequency ablation apparatus (100) acquires the multi-lead ECG signal by using body surface multi-leads in real time during the radio frequency ablation process, can learn the influence of radio frequency ablation on the blood supply to various sites of the heart, so as to analyze and monitor the heart function of the patient (200) in real time, and can realize automatic control over the radio frequency power according to an analysis result, such that excessive ablation or inadequate ablation is prevented, thereby ensuring the safety of an ablation surgery.

## Description

### TECHNICAL FIELD

The present application relates to the technical field of active medical devices, in particular to a radio frequency ablation apparatus.

### DESCRIPTION OF THE PRIOR ART

Hypertrophic cardiomyopathy is a common cardiovascular disease with autosomal dominant pattern, with clinical manifestation of dyspnea, precordial pain, fatigue, dizziness, fainting, palpitation, heart failure, and its main manifestation is one or more stages of left ventricle hypertrophy, the general diagnostic standard of which is having a thickness greater than or equal to 15mm. The treatment strategy for hypertrophic cardiomyopathy is to expand the left ventricular outflow tract to reduce the pressure gradient and alleviate the obstruction. The main methods include drug therapy, interventricular septum atherectomy, and interventricular septum alcohol ablation. Drug therapy has poor treatment effect or intolerance for some patients. Interventricular septum atherectomy involves certain risks and painful postoperative recovery for patients. Interventricular septum alcohol ablation has the risk of myocardial infarction caused by alcohol passing through branch vessels.

In recent years, hypertrophic cardiomyopathy has been treated with a radio frequency ablation method through a transcutaneous-intercostal-epicardium access, which uses high-frequency electric waves to cause the ions in the hypertrophic myocardial tissue cells to produce a thermal effect, with the local temperature reaching 90 to 100 °C, resulting in dehydration and necrosis of the hypertrophic myocardial cells and also achieving the effect of blocking the blood supply of hypertrophic myocardial tissue, so as to achieve the purpose of treatment. In order to ensure the safety of the ablation process and achieve the desired therapeutic effect, monitoring the patient's physiological signals and controlling the radio frequency energy are particularly important. However, the existing radio frequency ablation apparatuses usually control the output radio frequency energy by monitoring the ablation parameters of the ablation area during the ablation process, but it is impossible to know the impact of the ablation surgery on the blood supply to other sites of the heart. For example, when the temperature of the ablation site is high, it cannot reflect whether the inferior wall blood supply and electrical signal conduction are affected, and it may still lead to excessive ablation or inadequate ablation of myocardial tissue, so the safety and effectiveness are low.

### SUMMARY OF THE DISCLOSURE

The present disclosure provides a radio frequency ablation apparatus, which uses body surface leads to acquire ECG signals, can measure the vector information of the heart in all directions, and can obtain the impact of the ablation surgery on the blood supply to various sites of the heart according to the ECG signals, so as to better adjust the output of radio frequency power and ensure the safety of the ablation process and achieve the desired therapeutic effect.

The present disclosure provides a radio frequency ablation apparatus, which includes a radio frequency energy generation unit, an ablation component, a signal acquisition unit, and a control unit. The radio frequency energy generation unit is configured to generate and output radio frequency power required for radio frequency ablation. The ablation component is electrically connected to the radio frequency energy generation unit, and configured to transmit the radio frequency power output by the radio frequency energy generation unit to an ablation site of heart, so as to perform radio frequency ablation on the ablation site. The signal acquisition unit at least includes an ECG signal acquisition unit, and the ECG signal acquisition unit includes a plurality of electrodes, and the plurality of electrodes are configured to contact with a plurality of different body surface sites of a patient to acquire multi-lead ECG signal(s) represented by cardiac electrophysiological activity(s) acting on the body surface during radio frequency ablation. The control unit is electrically connected to the ECG signal acquisition unit and the radio frequency energy generation unit, respectively, and the control unit is configured to receive the multi-lead ECG signal(s) and control the radio frequency power output by the radio frequency energy generation unit according to the multi-lead ECG signal(s).

The radio frequency ablation apparatus provided by the present disclosure uses multi-leads on the body surface to acquire the multi-lead ECG signals in real time during the radio frequency ablation process, and can measure the vector information of the heart in all directions, thereby obtaining the impact of radio frequency ablation on the blood supply to various sites of the heart according to the acquired multi-lead ECG signals, so as to analyze and monitor the heart function of the patient in real time, and realize automatic control of the radio frequency power according to the analysis results, avoiding excessive ablation or inadequate ablation, ensuring the safety of ablation procedure and achieving the desired therapeutic effect.

### BRIEF DESCRIPTION OF DRAWINGS

In order to more clearly illustrate the technical solutions in the embodiments of the present disclosure or the prior art, the drawings will be briefly introduced in the following for describing the embodiments or the prior art. Apparently, the drawings in the following are only some embodiments of the present disclosure. For those skilled in the art, other drawings can be obtained according to these drawings without creative work.
FIG. 1 is a schematic diagram of functional modules of a radio frequency ablation apparatus according to an embodiment of the present disclosure.
FIG. 2 is a schematic diagram of functional modules of another radio frequency ablation apparatus according to an embodiment of the present disclosure.
FIG. 3 is a schematic diagram showing a radio frequency ablation apparatus in practice according to an embodiment of the present disclosure.
FIG. 4 is a schematic diagram of the ECG signal curve according to an embodiment of the present disclosure.
FIG. 5 is a flow chart of a radio frequency power control method according to an embodiment of the present disclosure.
FIG. 6 is a detailed flow chart of step 3108 in FIG. 5.
FIG. 7 is a detailed flow chart of step 3109 in FIG. 5.
FIG. 8 is a flow chart of another radio frequency power control method according to an embodiment of the present disclosure.

### List of reference signs

**[Table 1]**

| | |
|---|---|
| radio frequency ablation apparatus | 100 |
| operator | 18 |
| radio frequency ablation needle | 20 |
| signal acquisition unit | 21 |
| ECG signal acquisition unit | 211 |
| power acquisition unit | 212 |
| impedance acquisition unit | 213 |
| temperature acquisition unit | 214 |
| control unit | 22 |
| radio frequency energy generation unit | 23 |
| ablation component | 24 |
| human-computer interaction unit | 25 |
| patient | 200 |
| ECG electrode bundle | 30 |
| common electrode | 40 |
| target tissue area | 50 |
| electrode | RA, LA, LL, RL, C |
| step | 3101-3109, 3121-3124, 3131-3134, 4101-4114 |

The following specific embodiments will be illustrated in conjunction with the above-mentioned drawings for illustration of the present disclosure.

### DDESCRIPTION OF EMBODIMENTS

In the following, the technical solutions according to the embodiments of the present disclosure will be clearly and completely described with reference to the accompanying drawings according to the embodiments of the present disclosure. The drawings are used for illustrative purposes only, shown schematically, and should not be construed as limitations on the present disclosure. Apparently, the described embodiments are only some, not all, embodiments of the present disclosure. Based on the embodiments described in the present disclosure, all other embodiments obtained by the skilled persons in the art without creative efforts fall into the scope of protection of the present disclosure.

Unless otherwise defined, all technical and scientific terms used in the present disclosure have the same meaning as commonly understood by the skilled person in the art. The terms used in the description of the present disclosure are only for the purpose of describing specific embodiments, and are not intended to limit the present disclosure. In the following description, the terms such as 'module', 'component' or 'unit' for naming elements are only for description of the present disclosure and have no specific meanings. Therefore, 'module', 'component' or 'unit' can be used alternatively.

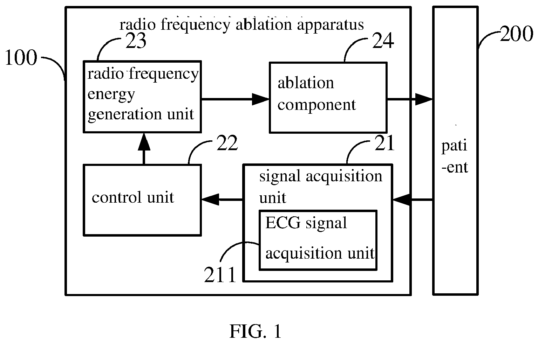

FIG. 1 is a block diagram of a radio frequency ablation apparatus according to an embodiment of the present disclosure. As shown in FIG. 1, the radio frequency ablation apparatus 100 includes a signal acquisition unit 21, a control unit 22, a radio frequency energy generation unit 23, and an ablation component 24.

The radio frequency energy generation unit 23 is configured to generate radio frequency power required for radio frequency ablation. The ablation unit 24 is electrically connected to the radio frequency energy generation unit 23, and configured to transmit the radio frequency power output by the radio frequency energy generation unit 23 to an ablation site of the heart of a patient 200, so as to perform radio frequency ablation on the ablation site. The control unit 22 is electrically connected to the signal acquisition unit 21 and the radio frequency energy generation unit 23 respectively. The signal acquisition unit 21 is configured to acquire physiological signals of the body of the patient 200 and transmit the physiological signals to the control unit 22. The control unit 22 is configured to receive the physiological signals acquired by the signal acquisition unit 21, analyze the physiological signals, and control the radio frequency power output by the radio frequency energy generation unit 23 according to the analysis result. The radio frequency power refers to the radio frequency energy output by the radio frequency energy generation unit 23 per unit time. The ablation component 24 transmitting the radio frequency power output by the radio frequency energy generation unit 23 to the ablation site can be understood as the ablation component 24 transmitting the radio frequency energy to the ablation site.

In this embodiment, the signal acquisition unit 21 includes at least an ECG signal acquisition unit 211. The ECG signal acquisition unit 211 includes a plurality of electrodes, and the plurality of electrodes are configured to contact with a plurality of different body surface sites of the patient 200 so as to acquire multi-lead ECG signals represented by the cardiac electrophysiological activities acting on the body surface during radio frequency ablation process. The control unit 22 is configured to receive the multi-lead ECG signals, and control the radio frequency power output by the radio frequency energy generation unit 23 according to the multi-lead ECG signals. It can be understood that which body surface sites the ECG electrodes are in contact with depends on which of three leads, five leads or twelve leads the ECG signal acquisition unit 211 uses.

The radio frequency ablation apparatus 100 according to the present disclosure acquires the multi-lead ECG signals in real time by using multiple leads on the body surface during the radio frequency ablation process, and can measure the vector information of the heart from various directions, so as to learn the influence of radio frequency ablation on blood supply to various sites of the heart according to the acquired multiple-lead ECG signals, and analyze and monitor the heart function of the patient 200 in real time , and can realize automatic control on the radio frequency power according to the analysis results to avoid excessive ablation or inadequate ablation, thereby ensuring the safety of ablation surgery and achieving the desired therapeutic effect.

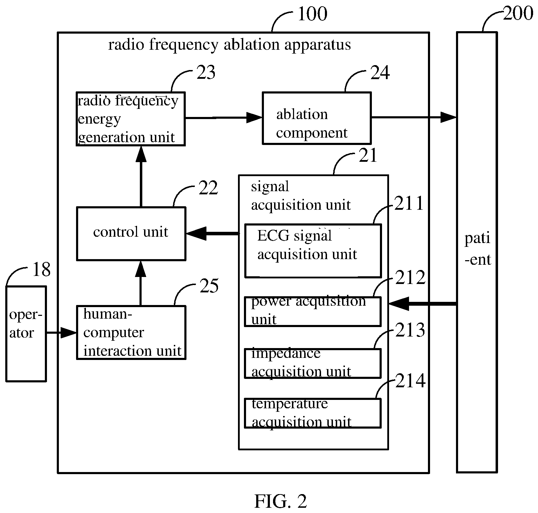

Further, referring to FIG. 2, the signal acquisition unit 21 further includes a power acquisition unit 212, an impedance acquisition unit 213 and / or a temperature acquisition unit 214, which are electrically connected to the control unit 22. The power acquisition unit 212 is configured to acquire the actual power signals of the radio frequency energy generation unit 23 acting on the ablation site, and transmit the actual power signals to the control unit 22 as the basic data for the control unit 22 to adjust the radio frequency power. The control unit 22 is configured to control the radio frequency power output by the radio frequency energy generation unit 23 according to the actual power signals.

The impedance acquisition unit 213 is configured to acquire the impedance signals of the ablation site, and transmit the impedance signals to the control unit 22 as the basic data for the control unit 22 to adjust the radio frequency power. The control unit 22 is configured to control the radio frequency power output by the radio frequency energy generation unit 23 according to the impedance signals.

The temperature acquisition unit 214 is configured to acquire the temperature signals of the ablation site, and transmit the temperature signals to the control unit 22 as the basic data for the control unit 22 to adjust the radio frequency power. The control unit 22 is configured to control the radio frequency power output by the radio frequency energy generation unit 23 according to the temperature signals.

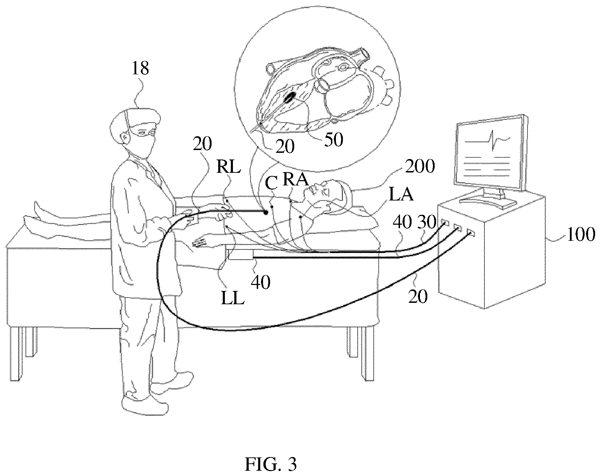

FIG. 3 is a schematic diagram showing the radio frequency ablation apparatus 100 in practice according to an embodiment of the present disclosure. The ablation component 24 can include but not limited to a radio frequency ablation catheter or a radio frequency ablation needle. In the case where the ablation component 24 includes a radio frequency ablation needle, it can be used for ablation of interventricular septal hypertrophy. In the case where the ablation component 24 includes a radio frequency ablation catheter, it can be used for ablation of the inner wall of the myocardium. Here, the radio frequency ablation apparatus 100 is introduced by taking the ablation component 24 including the radio frequency ablation needle 20 as an example. Specifically, the radio frequency ablation needle 20 reaches the target tissue area 50 (i.e., the ablation site) through a transcutaneous-intercostal-epicardium access under the guidance of ultrasound. The control unit 22 controls the radio frequency energy generation unit 23 to output the radio frequency power acting on the target tissue area 50 through the radio frequency ablation needle 20, forming an ablation area at the space around the exposed non-insulated area of the radio frequency ablation needle 20. The tissue cells in the ablation area become dehydrated and necrotic after ablation, and finally the myocardium becomes thinner, thereby achieving the therapeutic effect.

Further, the temperature acquisition unit 214, such as a thermocouple, is located at the needle tip of the radio frequency ablation needle 20, and configured to acquire the temperature in the target tissue region 50 in real time. The radio frequency ablation apparatus 100 supports electrocardiogram (ECG) three-lead connection, five-lead connection and twelve-lead connection, etc., without specific limitation here. In this embodiment, the radio frequency ablation apparatus 100 is introduced by taking ECG five -lead connection as an example. As shown in FIG. 3, the ECG electrode bundle 30 includes 5 electrode wires in total. The electrode RA is placed on the upper right, specifically, at the first intercostal space on the right sternal border along the midclavicular line; the electrode LA is placed on the upper left, specifically, at the first intercostal space on the left sternal border along the midclavicular line; the electrode LL is placed on the lower left, specifically, at the level of the xiphoid process along the left midclavicular line; electrode RL is placed on the lower right, specifically, at the level of the xiphoid process along the right midclavicular line; electrode C is placed in the middle, specifically, at the fourth intercostal space on the left sternal border or at a corresponding position as needed which can be any one of C1-C6 where the electrode C is placed according to the needs of the operator 18. The electrode RA, the electrode LA, the electrode LL, the electrode RL and the electrode C constitute the ECG signal acquisition unit 211 for acquiring and monitoring the multi-lead ECG signals in real time. The common electrode 40 is connected to an appropriate position on the body surface of the patient 200, such as the thigh of the patient 200, using a negative plate connecting wire. The common electrode 40 serves as both the negative electrode in the radio frequency ablation circuit and the common electrode of the ECG electrodes.

In this embodiment, the physiological signals include at least the multi-lead ECG signals. The control unit 22 analyzes the cardiac electrophysiological activities according to the received multi-lead ECG signals, and controls the radio frequency power output by the radio frequency energy generation unit 23 according to the analysis result.

It can be understood that ECG signal monitoring during the radio frequency ablation process is different from the monitoring in care unit or physical examination in that more attention is paid to real-time heart rate and real-time ST segment amplitude during ablation. Therefore, in radio frequency ablation, monitoring the cardiac electrophysiological activities includes monitoring at least one of the real-time heart rate and the real-time ST segment amplitude.

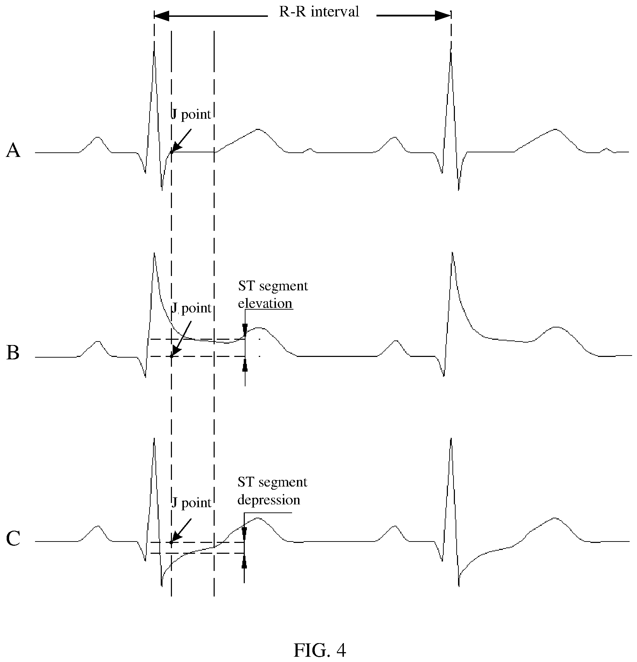

FIG. 4 is a schematic diagram of the ECG signal curve according to the embodiment of the present disclosure. In this embodiment, the control unit 22 is further configured to analyze at least one of the real-time heart rate and the real-time ST segment amplitude of the multi-lead ECG signals.

Specifically, the control unit 22 is configured to calculate the real-time heart rate hr_x (heart rate unit: bpm (beat per minute), the number of heart beats per minute) of the patient 200 according to the multi-lead ECG signals. The heart rate value can be calculated according to the heart rate calculation formula: hr=60/t_{R-R}, wherein t_{R-R} represents the time interval between two adjacent R wave peaks in a R-R interval in seconds.

The control unit 22 is further configured to monitor the real-time ST segment amplitude st_x (in mV) according to the multi-lead ECG signals. The ST segment represents the ventricular activation. When the ST segment is elevated, hyperacute or acute myocardial infarction is prone to occur, and when the ST segment is depressed, myocardial ischemia is prone to occur.

In this embodiment, the control unit 22 is further configured to determine the risk level of arrhythmia according to the analysis results of the real-time heart rate hr_x and / or the real-time ST segment amplitude st_x, and issue an instruction to stop outputting the radio frequency power to control the radio frequency energy generation unit 23 to stop outputting the radio frequency power when the risk level of arrhythmia is high.

Specifically, the control unit 22 is configured to calculate the real-time heart rate hr_x of the patient 200 during the ablation process according to the multi-lead ECG signals, and determine whether the real-time heart rate hr_x does not fall within the heart rate threshold range, wherein the heart rate threshold includes a heart rate upper limit hr_max and a heart rate lower limit hr_min, which can be set or preset and stored in the control unit 22 by the operator 18.

The control unit 22 is further configured to determine that the risk level of arrhythmia is high when the real-time heart rate hr_x does not fall within the heart rate threshold range, that is, hr_x ≥ hr_max or hr_x ≤ hr_min, and control the radio frequency energy generation unit 23 to stop outputting the radio frequency power.

Optionally, the control unit 22 is further configured to calculate the change rate of heart rate hr_rate according to the multi-lead ECG signals, and determine whether the change rate of heart rate hr_rate is greater than a first change rate threshold of heart rate. The first change rate threshold of heart rate can be set or preset and stored in the control unit 22 by the operator 18, for example, it can be set to 50%. Specifically, the change rate of heart rate hr_rate is the change rate of the real-time heart rate hr_x relative to the initial heart rate hr_init , and its calculation formula is:

hr_rate=(|hr_x-hr_init|)/hr_init, where hr_init is the initial heart rate, which can be obtained from multi-lead ECG signals obtained before radio frequency ablation, and hr_x is the monitored real-time heart rate during radio frequency ablation.

The control unit 22 is further configured to determine that the risk level of arrhythmia is high when the change rate of heart rate hr_rate is greater than the first change rate threshold of heart rate, for example, when hr_rate > 50%, and control the radio frequency energy generation unit 23 to stop outputting the radio frequency power.

Optionally, the control unit 22 is further configured to calculate the variation rate of heart rate hr_fd according to the multi-lead ECG signals, and determine whether the absolute value of the variation rate of heart rate hr_fd is not less than the variation rate threshold of heart rate hr_v. Specifically, the variation rate of heart rate hr_fd is the variation rate of the real-time heart rate hr_x relative to the initial heart rate hr_init, and its value is obtained by doing a first derivation on the monitored real-time heart rate hr_x. The variation rate threshold of heart rate hr_v can be set or preset and stored in the control unit 22 by the operator 18.

The control unit 22 is further configured to determine that the risk level of arrhythmia is high when the absolute value of the variation rate of heart rate hr_fd is not less than the variation rate threshold of heart rate hr_v, that is, |hr_fd| ≥ hr_v, and control the radio frequency energy generation unit 23 to stop outputting the radio frequency power.

Optionally, the control unit 22 is further configured to obtain the real-time ST segment amplitude st_x according to the multi-lead ECG signals and calculate the offset of ST segment amplitude st_sft, and determine whether the offset of ST segment amplitude st_sft is not less than the offset threshold of ST segment amplitude. The offset threshold of ST segment amplitude can be set or preset and stored in the control unit 22 by the operator 18, for example, it can be set to 0.1 mV. Specifically, the offset of ST segment amplitude st_sft is the offset of the real-time ST segment amplitude st_x relative to the initial ST segment amplitude st_init, and its calculation formula is:

st_sft = |st_x-st_init|, where st init is the initial ST segment amplitude, which can be obtained from multi-lead ECG signals obtained before radio frequency ablation, and st_x is the real-time ST segment amplitude. Alternatively, the offset of ST segment amplitude st_sft can be obtained by calculating the difference between the amplitude at the ST segment and the amplitude at the J point in the same waveform period after filtering the baseline drift, wherein the amplitude at the J point is equal to the initial ST segment amplitude. As shown in FIG. 4, waveform B has ST segment elevation relative to the J point, and waveform C has ST segment depression relative to the J point. The baseline drift refers to the fluctuation of the waveform relative to the horizontal baseline during the signal measurement process.

The control unit 22 is further configured to determine that the risk level of arrhythmia is high when the offset of ST segment amplitude st_sft is not less than the offset threshold of ST segment amplitude, for example, when st_sft ≥ 0.1mV, and control the radio frequency energy generation unit 23 to stop outputting the radio frequency power.

Optionally, the control unit 22 is further configured to calculate the variation rate of ST segment amplitude st_fd according to the multi-lead ECG signals, and determine whether the absolute value of the variation rate of ST segment amplitude st_fd is not less than the variation rate threshold of ST segment amplitude st_v. Specifically, the variation rate of ST segment amplitude st_fd is the variation rate of the real-time ST segment amplitude st_x relative to the initial ST segment amplitude st_init, and its value is obtained by doing a first derivation on the monitored real-time ST segment amplitude st_x. The variation rate threshold of ST segment amplitude st_v can be set or preset and stored in the control unit 22 by the operator 18.

The control unit 22 is further configured to determine the risk level of arrhythmia is high when the absolute value of the variation rate of ST segment amplitude st_fd is not less than the variation rate threshold of ST segment amplitude st_v, that is, |st_fd| ≥ st_v, and control the radio frequency energy generation unit 23 to stop outputting the radio frequency power.

It can be understood that the control unit 22 is configured to determine whether the risk level of arrhythmia is high or not according to the analysis results of the real-time heart rate hr_x and the real-time ST segment amplitude st_x.

Optionally, when it is determined that the risk level of arrhythmia is not high according to the analysis results of the real-time heart rate hr_x and / or the real-time ST segment amplitude st_x, the control unit 22 is further configured to determine whether the change rate of heart rate hr_rate is greater than a second change rate threshold of heart rate, and control the radio frequency energy generation unit 23 to pause outputting the radio frequency power or reduce the output radio frequency power when the change rate of heart rate hr_rate is greater than the second change rate threshold of heart rate. The second change rate threshold of heart rate is smaller than the first change rate threshold of heart rate, and can be set or preset and stored in the control unit 22 by the operator 18, for example it can be set to 20%.

Optionally, when it is determined that the risk level of arrhythmia is not high according to the analysis results of the real-time heart rate hr_x and / or the real-time ST segment amplitude st_x and the change rate of heart rate hr_rate is not greater than the second change rate threshold of heart rate, the control unit 22 is further configured to calculate the change rate of ST segment amplitude st_rate according to the multi-lead ECG signals and determine whether the change rate of ST segment amplitude st_rate is greater than the change rate threshold of ST segment amplitude st_d which can be set or preset and stored in the control unit 22 by the operator 18. Specifically, the change rate of ST segment amplitude st_rate is the change rate of the real-time ST segment amplitude st_x relative to the initial ST segment amplitude st_init, and its calculation formula is:

st_rate =(|st_x- st_init|)/st_init, where st_x is the real-time ST segment amplitude, and st init is the initial ST segment amplitude.

The control unit 22 is further configured to control the radio frequency energy generation unit 23 to pause outputting the radio frequency power or reduce the output radio frequency power when the change rate of ST segment amplitude st_rate is greater than the change rate threshold of ST segment amplitude st_d, that is, st_rate > st_d.

In this embodiment, the ECG signal acquisition unit 211 acquires the multi-lead ECG signals once in each measurement period. For example, the measurement period is 1 second, that is, the ECG signal acquisition unit 211 acquires the multi-lead ECG signal once per second. Therefore, the control unit 22 can analyze the real-time heart rate hr_x and / or the real-time ST segment amplitude st_x according to each multi-lead ECG signal.

Further, the radio frequency ablation apparatus 100 further includes a first counting module (not shown in the figure) and a second counting module (not shown in the figure), both of which are electrically connected to the control unit 22. The first counting module is configured to count the number of times that the change rate of heart rate hr_rate is continuously greater than the second change rate threshold of heart rate. The second counting module is configured to count the number of times that the change rate of ST segment amplitude st_rate is continuously greater than the change rate threshold of ST segment amplitude st_d. The initial counting values of the first counting module and the second counting module are both zero.

The control unit 22 is further configured to control the count value of the first counting module to add one when the change rate of heart rate hr_rate is greater than the second change rate threshold of heart rate, and determine whether the count value of the first counting module is greater than a first count threshold. The first count threshold can be set or preset and stored in the control unit 22 by the operator 18, for example, it can be set to 1.

The control unit 22 is further configured to issue an instruction to pause outputting radio frequency power when the count value of the first counting module is greater than the first count threshold, so as to control the radio frequency energy generation unit 23 to pause outputting the radio frequency power, and control the first counting module to be cleared to zero.

The control unit 22 is further configured to issue an instruction to reduce the radio frequency power when the count value of the first counting module is not greater than the first count threshold, so as to control the radio frequency energy generation unit 23 to reduce the output radio frequency power.

Optionally, the control unit 22 is further configured to control the count value of the second counting module to add one when the change rate of ST segment amplitude st_rate is greater than the change rate threshold of ST segment amplitude st_d, and determine whether the count value of the second counting module is greater than a second count threshold. The second count threshold can be set or preset and stored in the control unit 22 by the operator 18, for example, the second count threshold can be set to 1.

The control unit 22 is further configured to issue the instruction to pause outputting radio frequency power when the count value of the second counting module is greater than the second count threshold, so as to control the radio frequency energy generation unit 23 to pause outputting the radio frequency power, and control the second counting module to be cleared to zero.

The control unit 22 is further configured to issue the instruction to reduce the radio frequency power when the count value of the second counting module is not greater than the second count threshold, so as to control the radio frequency energy generation unit 23 to reduce the output radio frequency power.

It should be noted that stopping outputting the radio frequency power means that the radio frequency energy generation unit 23 stops outputting the radio frequency power, and the radio frequency ablation process does not continue. Pausing outputting the radio frequency power means that the radio frequency energy generation unit 23 pauses outputting the radio frequency power, and after a period of time, the operator 18 manually controls the control unit 22 to issue a start instruction to control the radio frequency energy generation unit 23 to continue outputting the radio frequency power, or by setting a pause time threshold, the control unit 22 automatically issues a start instruction to control the radio frequency energy generation unit 23 to continue outputting the radio frequency power when the pause time reaches the pause time threshold, wherein the radio frequency power that is output continuously can be the same as the radio frequency power that was output before the pause. Reducing the output radio frequency power means that the radio frequency energy generation unit 23 outputs a radio frequency power that is smaller than the original radio frequency power and greater than zero according to a preset reduction rule, for example, a reduced radio frequency power being the half of the original radio frequency power.

In this embodiment, the radio frequency energy generation unit 23 is further configured to receive the control instruction issued by the control unit 22, adjust the output radio frequency power, and then transmit the adjusted radio frequency power to the ablation component 24. The ablation component 24 is configured to apply the received radio frequency power to the patient 200 for ablation treatment.

In this embodiment, the radio frequency ablation apparatus 100 is configured to monitor the multi-lead ECG signals represented by the cardiac electrophysiological activities acting on the body surface of the patient 200 through the signal acquisition unit 21 in surgery. The control unit 22 is configured to analyze and process the multi-lead ECG signals so as to control the radio frequency power output by the radio frequency energy generation unit 23, and can analyze the conditions of different sites of the heart of the patient 200 more comprehensively in surgery and then perform corresponding control, thereby ensuring the safety of ablation surgery and achieving the desired therapeutic effect.

Optionally, the control unit 22 is further configured to obtain the impedance signals of the ablation site, and issue an instruction to pause outputting the radio frequency power to control the radio frequency energy generation unit 23 to pause outputting the radio frequency power when the impedance value R of the impedance signal satisfies any one of a first preset condition and a second preset condition. Specifically, the first preset condition is: R > K1 ×Rmin1, and the second preset condition is: R > K2×Rmin2, where K1 and K2 are proportional coefficients, and K1 < K2, Rmin1 is the lowest impedance monitored in a preset period during the period of recording the ablation time, wherein the preset period is the period closest to the current moment and with a preset time length, and Rmin2 is the lowest impedance monitored during the period of recording the ablation time.

For example, assuming that the preset time length is 20s and starting timekeeping at the beginning of the ablation, if the currently recorded ablation time is 10s, then Rmin1 and Rmin2 are both the lowest impedances monitored within 0 to 10s. If the currently recorded ablation time is 35s, then Rmin1 is the lowest impedance monitored within 15 to 35s, while Rmin2 is the lowest impedance monitored within 0 to 35s. It should be noted that the ablation time in this embodiment does not include the time when the radio frequency energy generation unit 23 pauses outputting the radio frequency power.

Optionally, the control unit 22 is further configured to obtain the temperature signals of the ablation site, and issue an instruction to stop outputting the radio frequency power to control the radio frequency energy generation unit 23 to stop outputting the radio frequency power when the temperature value of the temperature signal is greater than the temperature upper limit.

Optionally, the control unit 22 is further configured to obtain the actual power signal acting on the ablation site, and control the radio frequency energy generation unit 23 to adjust the output radio frequency power to a set power value when the power value of the actual power signal is inconsistent with the set power value.

In some embodiments, the signal acquisition unit 21 can include at least one of the power acquisition unit 212, the impedance acquisition unit 213, and the temperature acquisition unit 214, which are not specifically limited here.

Referring to FIG. 2 again, the radio frequency ablation apparatus 100 further includes a human-computer interaction unit 25 electrically connected to the control unit 22. The human-computer interaction unit 25 can be provided with an input unit and a display unit. The input unit can be a mechanical button, a mechanical knob, a touch button, or a touch screen that can display virtual buttons, etc., and is configured to acquire the ablation parameters set by the operator 18 and transmit the set ablation parameters to the control unit 22. The control unit 22 is further configured to control the radio frequency power output by the radio frequency energy generation unit 23 according to the set ablation parameters. Specifically, the ablation parameters include the initial radio frequency power and ablation time determined by the operator 18 according to the size of the ablation site before the surgery, and the expected radio frequency power among other parameters adjusted by the operator 18 according to the condition of the patient 200 during the surgery.

In some embodiments, the input unit can further include a start button, a pause button, and a stop button. Further, the control unit 22 is further configured to issue a start instruction when the start button is pressed, issue an instruction to pause outputting the radio frequency power when the pause button is pressed, and issue an instruction to stop outputting the radio frequency power when the stop button is pressed. It can be understood that the start button and the pause button can be the same button, and when this button is pressed, the control unit 22 alternately issues the start instruction and the instruction to pause outputting the radio frequency power.

The display unit can be a liquid crystal display (LCD), organic light-emitting diode (OLED ), etc., configured to display the ablation parameters (such as ablation time, initial radio frequency power), the real-time signal values (such as real-time radio frequency power value, real-time impedance value, ablation duration) acquired by the signal acquisition unit 21 during the surgery and signal waveforms (such as multi-lead ECG signal waveform) among others, thereby displaying the real-time ablation status. Therefore, the operator 18 can know the ablation detail by observing various data displayed by the display unit, and adjust the output of the radio frequency power by operating the input unit, ensuring the safety of the ablation surgery and achieving the desired therapeutic effect.

In addition to analyzing the multi-lead ECG signals and controlling the radio frequency power according to the analysis results, the radio frequency ablation apparatus 100 according to this embodiment further includes the temperature acquisition unit 214 and the impedance acquisition unit 213. The control unit 22 is further configured to determine whether the temperature signal and the impedance signal acquired by the temperature acquisition unit 214 and the impedance acquisition unit 213 respectively satisfy the corresponding preset conditions, so as to control the radio frequency power output by the radio frequency energy generation unit 23. Due to the monitoring on the temperature and the impedance, the physical condition of the patient 200 during the surgery can be known more comprehensively, and then corresponding control can be performed to ensure the safety of the ablation surgery and achieving the desired therapeutic effect.

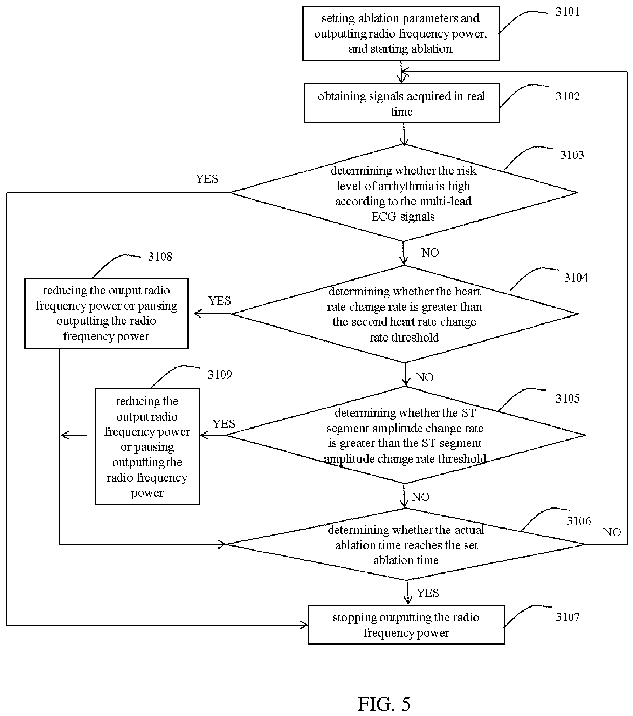

The present disclosure further provides a radio frequency power control method based on multi-lead ECG signals, which is implemented by the above radio frequency ablation apparatus 100. FIG. 5 is a flow chart of a radio frequency power control method according to an embodiment of the present disclosure. It should be noted that the radio frequency power control method described in the embodiment of the present disclosure is not limited to the steps and orders in the flow chart shown in FIG. 5. Depending on various requirements, steps shown in the flow chart can be added, removed, or changed in order. As shown in FIG. 5, the radio frequency power control method based on multi-lead ECG signals includes the following steps:

Step 3101, setting ablation parameters and outputting radio frequency power, and starting ablation.

In this embodiment, the ablation parameters at least include parameters such as ablation time and initial radio frequency power. After the parameters are set, the radio frequency ablation apparatus 100 outputs the radio frequency power according to the ablation parameters, starts the ablation, and clocking the actual ablation time.

Step 3102, obtaining signals acquired in real time.

The signals at least include multi-lead ECG signals. Specifically, the multi-lead ECG signals can be three-lead, five-lead or twelve-lead ECG signals, which are not limited here.

Step 3103, determining whether the risk level of arrhythmia is high according to the multi-lead ECG signals.

If the risk level of arrhythmia is high, step 3107 is performed. Otherwise, step 3104 is performed.

Specifically, when the preset condition is satisfied according to the comprehensive judgment of the multi-lead ECG signals, it can be determined that the risk level of arrhythmia is high, and when the preset condition is not satisfied, it can be determined that the risk level of arrhythmia is not high. Further, the preset condition can be set to at least one of the following.

The real-time heart rate hr_x does not fall within the heart rate threshold range, wherein the heart rate threshold includes a heart rate upper limit hr_max and a heart rate lower limit hr_min, that is, hr_x ≥ hr_max or hr_x ≤ hr_min.

The variation rate of heart rate hr_rate is greater than the first variation rate threshold of heart rate.

The absolute value of the variation rate of heart rate hr_fd is not less than the variation rate threshold of heart rate hr_v.

The offset of ST segment amplitude st_sft is not less than the offset threshold of ST segment amplitude.

The absolute value of the variation rate of ST segment amplitude st_fd is not less than the variation rate threshold of ST segment amplitude st_v.

It should be noted that the real-time heart rate hr_x is the real-time heart rate of the patient 200 during the ablation process calculated according to the multi-lead ECG signals (heart rate unit: bpm (beat per minute), the number of heart beats per minute). The heart rate value can be calculated according to the heart rate calculation formula: hr=60/t_{R- R}, wherein t_{R-R} represents the time interval between two adjacent R wave peaks in a R-R interval in seconds.

The change rate of heart rate hr_rate is the change rate of the real-time heart rate hr_x relative to the initial heart rate hr init , and its calculation formula is:

hr_rate=(|hr_x-hr_init|)/hr_init, where hr_init is the initial heart rate, which can be obtained from multi-lead ECG signals obtained before radio frequency ablation, and hr_x is the monitored real-time heart rate during radio frequency ablation.

The variation rate of heart rate hr_fd is the variation rate of the real-time heart rate hr_x relative to the initial heart rate hr_init, and its value is obtained by doing a first derivation on the monitored real-time heart rate hr_x.

The offset of ST segment amplitude st_sft is the offset of the real-time ST segment amplitude st_x relative to the initial ST segment amplitude st_init, and its calculation formula is:

st_sft = |st_x-st_init|, where st init is the initial ST segment amplitude, which can be obtained from multi-lead ECG signals obtained before radio frequency ablation, and st_x is the real-time ST segment amplitude. Alternatively, the offset of ST segment amplitude st_sft can be obtained by calculating the difference between the amplitude at the ST segment and the amplitude at the J point in the same waveform period after filtering the baseline drift, wherein the amplitude at the J point is equal to the initial ST segment amplitude. As shown in FIG. 4, waveform B has ST segment elevation relative to the J point, and waveform C has ST segment depression relative to the J point. The baseline drift refers to the fluctuation of the waveform relative to the horizontal baseline during the signal measurement process.

The variation rate of ST segment amplitude st_fd is the variation rate of the real-time ST segment amplitude st_x relative to the initial ST segment amplitude st_init, and its value is obtained by doing a first derivation on the monitored real-time ST segment amplitude st_x.

Step 3104, determining whether the change rate of heart rate is greater than the second change rate threshold of heart rate.

If the change rate of heart rate is greater than the second change rate threshold of heart rate, step 3108 is performed. Otherwise, step 3105 is performed.

Step 3105, determining whether the change rate of ST segment amplitude is greater than the change rate threshold of ST segment amplitude.

If the change rate of ST segment amplitude is greater than the change rate threshold of ST segment amplitude, step 3109 is performed. Otherwise, step 3106 is performed.

Step 3106, determining whether the actual ablation time reaches the set ablation time.

If the actual ablation time has reached the set ablation time, step 3107 is performed. Otherwise, step 3102 is performed again to continue the ablation.

Step 3107, stopping outputting the radio frequency power.

Step 3108, reducing the output radio frequency power or pause outputting the radio frequency power.

Step 3109, reducing the output radio frequency power or pause outputting the radio frequency power.

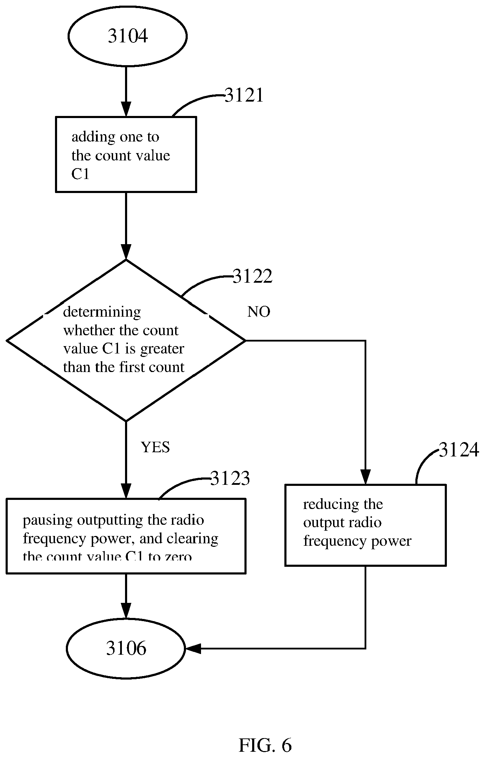

Referring to FIG. 6, FIG. 6 is a detailed flow chart of step 3108 for describing the above embodiment in more detail.

As shown in FIG. 6, after it is determined in step 3104 that the change rate of heart rate is greater than the second change rate threshold of heart rate, step 3121 is performed.

Step 3121, adding one to the count value C1. It can be understood that the initial value of the count value C1 of the first counting module is zero.

Step 3122, determining whether the count value C1 is greater than the first count threshold. If the count value C1 is greater than the first count threshold, step 3123 is performed. Otherwise, step 3124 is performed.

Step 3123, pause outputting the radio frequency power, and clearing the count value C1 to zero. Optionally, when the output of the radio frequency power is paused, the pause time is recorded and the recording of the actual ablation time is paused simultaneously. If the pause time reaches the pause time threshold, the output of the radio frequency power is continued, and the recording of the actual ablation time is continued. The pause time threshold can be set by the operator 18. Optionally, after the ablation is paused for a period of time, the operator 18 can manually control the control unit 22 to issue a start instruction to control the radio frequency energy generation unit 23 to continue outputting the radio frequency power. The radio frequency power that is output continuously can be the same as the radio frequency power that was output before the pause. Step 3106 is performed after step 3123 is performed.

Step 3124, reducing the output radio frequency power. Step 3106 is performed after step 3124 is performed.

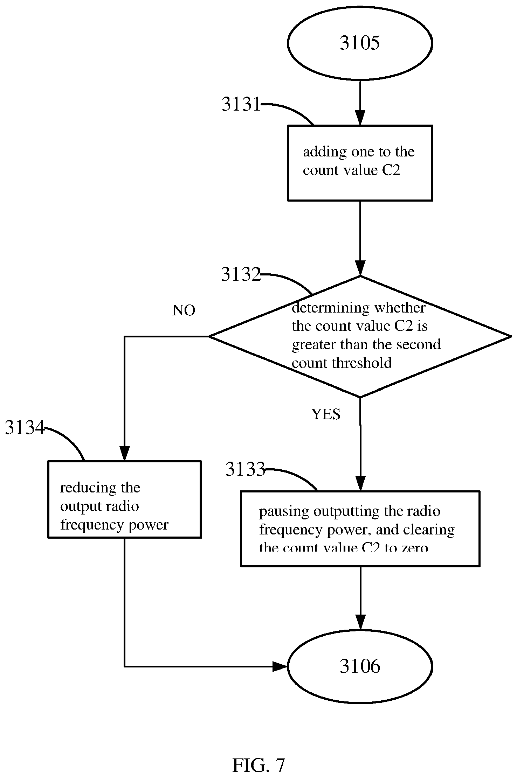

Referring to FIG. 7, FIG. 7 is a detailed flow chart of step 3109 for describing the above embodiment in more detail.

As shown in FIG. 7, after it is determined in step 3105 that the change rate of ST segment amplitude is greater than the change rate threshold of ST segment amplitude, step 3131 is performed.

Step 3131, adding one to the count value C2. It can be understood that the initial value of the count value C2 of the second counting module is zero.

Step 3132, determining whether the count value C2 is greater than the second count threshold. If the count value C2 is greater than the second count threshold, step 3133 is performed. Otherwise, step 3134 is performed.

Step 3133, pause outputting the radio frequency power, and clearing the count value C2 to zero. Optionally, when the output of the radio frequency power is paused, the pause time is recorded and the recording of the actual ablation time is paused simultaneously. If the pause time reaches the pause time threshold, the output of the radio frequency power is continued, and the recording of the actual ablation time is continued. The pause time threshold can be set by the operator 18. Optionally, after the ablation is paused for a period of time, the operator 18 can manually control the control unit 22 to issue a start instruction to control the radio frequency energy generation unit 23 to continue outputting the radio frequency power. The radio frequency power that is output continuously can be the same as the radio frequency power that was output before the pause. Step 3106 is performed after step 3133 is performed.

Step 3134, reducing the output radio frequency power. Step 3106 is performed after step 3134 is performed.

In the present disclosure, the heart rate threshold, the first change rate threshold of heart rate, the second change rate threshold of heart rate, the variation rate threshold of heart rate, the offset threshold of ST segment amplitude, the variation rate threshold of ST segment amplitude, the first count threshold, the second count threshold and the change rate threshold of ST segment amplitude are not specifically defined. It can be understood that the operator 18 can preset appropriate thresholds according to the actual surgery practice. For example, the first change rate threshold of heart rate can be set to 50%, and the offset threshold of ST segment amplitude can be set to 0.1 mV.

The radio frequency power control method according to the present disclosure analyzes the real-time heart rate hr_x and the real-time ST segment amplitude st_x according to the acquired multi-lead ECG signals. When the above two information parameters meet the corresponding preset conditions, the radio frequency energy generation unit 23 is automatically controlled to reduce the output radio frequency power accordingly, or the output of the radio frequency power is paused or stopped, so as to control the radio frequency power in real time. The response speed is fast and the control is more accurate, ensuring the safety of the ablation surgery and achieving the desired therapeutic effect.

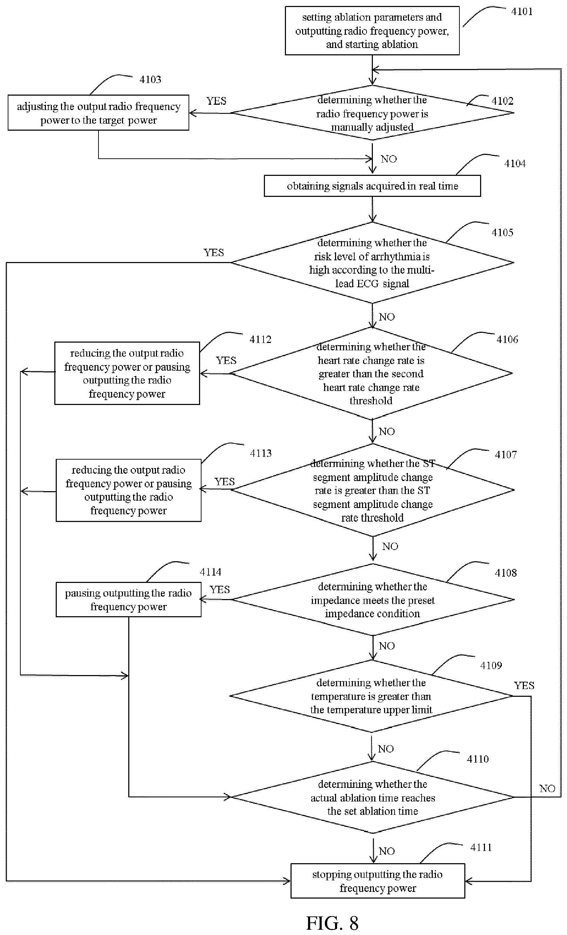

Referring to FIG. 8, the present disclosure further provides another radio frequency power control method based on multi-lead ECG signals, which is implemented by the aforementioned radio frequency ablation apparatus 100. FIG. 8 is a flowchart of a radio frequency power control method according to the embodiment of the present disclosure. It should be noted that the radio frequency power control method described in the embodiment of the present disclosure is not limited to the steps and orders in the flowchart shown in FIG. 8. Depending on various requirements, steps shown in the flow chart can be added, removed, or changed in order. As shown in FIG. 8, the radio frequency power control method based on multi-lead ECG signals includes the following steps:

Step 4101, setting ablation parameters and outputting radio frequency power, and starting ablation.

In this embodiment, the ablation parameters at least include parameters such as ablation time, preset impedance condition, temperature upper limit, and initial radio frequency power. After the parameters are set, the radio frequency ablation apparatus 100 outputs the radio frequency power according to the ablation parameters, starts the ablation, and clocking the actual ablation time.

Step 4102, determining whether the radio frequency power is manually adjusted.

By monitoring the human-computer interaction unit 25 in real time, it can be determined whether the radio frequency power is manually adjusted. If yes, step 4103 is performed. Otherwise, step 4104 performed.

Step 4103, adjusting the output radio frequency power to the target power.

The human-computer interaction unit 25 transmits the obtained target power value to the control unit 22, and the control unit 22 controls the radio frequency energy generation unit 23 to output radio frequency power with the target power value.

Step 4104, obtaining signals acquired in real time.

The signals at least include multi-lead ECG signals, temperature signals, and impedance signals. Specifically, the multi-lead ECG signals can be three-lead, five-lead or twelve-lead ECG signals, which are not limited here; the temperature signals refers to the temperature signals of the ablation site; and the impedance signals refers to the impedance signals of the ablation site.

Step 4105, determining whether the risk level of arrhythmia is high according to the multi-lead ECG signal.

If the risk level of arrhythmia is high, step 4111 is performed. Otherwise, step 4106 is performed.

Step 4106, determining whether the change rate of heart rate is greater than the second change rate threshold of heart rate.

If the change rate of heart rate is greater than the second change rate threshold of heart rate, step 4112 is performed. Otherwise, step 4107 is performed.

Step 4107, determining whether the change rate of ST segment amplitude is greater than the change rate threshold of ST segment amplitude.

If the change rate of ST segment amplitude is greater than the change rate threshold of ST segment amplitude, step 4113 is performed. Otherwise, step 4108 is performed.

Step 4108, determining whether the impedance meets the preset impedance condition.

If the impedance meets the preset impedance condition, step 4114 is performed. Otherwise, step 4109 is performed.

Specifically, the impedance is obtained from the impedance signals, and when the impedance value R satisfies any one of the first preset condition and the second preset condition, it is determined that the impedance satisfies the preset impedance condition. The first preset condition is: R > K1×Rmin1, and the second preset condition is: R > K2×Rmin2, where K1 and K2 are proportional coefficients, and K1 < K2, Rmin1 is the lowest impedance monitored in a preset period during the period of recording the ablation time, wherein the preset period is the period closest to the current moment and with a preset time length, and Rmin2 is the lowest impedance monitored during the period of recording the ablation time.

For example, assuming that the preset time length is 20s and starting timekeeping at the beginning of the ablation, if the currently recorded ablation time is 10s, then Rmin1 and Rmin2 are both the lowest impedances monitored within 0 to 10s. If the currently recorded ablation time is 35s, then Rmin1 is the lowest impedance monitored within 15 to 35s, while Rmin2 is the lowest impedance monitored within 0 to 35s. It should be noted that the ablation time in this embodiment does not include the time when the radio frequency energy generation unit 23 pauses outputting the radio frequency power.

Step 4109, determining whether the temperature is greater than the temperature upper limit.

If the temperature is greater than the temperature upper limit, step 4111 is performed. Otherwise, step 4110 is performed. Specifically, the temperature is obtained from the temperature signals.

Step 4110, determining whether the actual ablation time reaches the set ablation time.

If the actual ablation time has reached the set ablation time, step 4111 is performed. Otherwise, step 4102 is performed again.

Step 4111, stopping outputting the radio frequency power.

Step 4112, reducing the output radio frequency power or pause outputting the radio frequency power.

Step 4113, reducing the output radio frequency power or pause outputting the radio frequency power.

Step 4114, pause outputting the radio frequency power.

The specific technical details of steps 4101, 4104-4107, and 4110-4113 according to this embodiment can be referred to the relevant technical details of steps 3101-3109 according to the embodiment shown in FIG. 5, which would not be repeated here.

The radio frequency power control method according to the present embodiment comprehensively analyzes the real-time heart rate hr_x and the real-time ST segment amplitude st_x according to the acquired multi-lead ECG signals, and also analyzes the acquired temperature signals and impedance signal. When the real-time heart rate hr_x, the real-time ST segment amplitude st_x, temperature and impedance meet the corresponding preset conditions, the radio frequency energy generation unit 23 is automatically controlled to reduce the output radio frequency energy accordingly, or pause or stop outputting the radio frequency energy, so as to control the radio frequency power in real time. Based on the multi-lead ECG signals, the temperature and impedance are further monitored, so that the physical condition of the patient 200 during the surgery can be more fully known, thereby increasing the accuracy and timeliness of power control, further improving the safety, and achieving the desired therapeutic effect of ablation.

It will be apparent to those skilled in the art that the present disclosure is not limited to the details of the exemplary embodiments described above, but that the present disclosure can be implemented in other specific forms without departing from the spirit or essential features of the present disclosure. Therefore, the embodiments should be regarded as exemplary and not for limitation purpose. The scope of the disclosure is defined by the appended claims rather than the foregoing description, and the scope of the present disclosure is defined by the appended claims rather than by the foregoing description. All changes within the meaning and scope of equivalents of the claims are included in the present disclosure. Any reference sign in a claim should not be construed as limiting the claim concerned. In addition, it is apparent that the word "comprising" does not exclude other elements or steps, and the singular does not exclude the plural.

Finally, it should be noted that the above embodiments are only configured to illustrate the technical solutions of the present disclosure rather than limit the same. Although the present disclosure has been described in detail with reference to the above preferred embodiments, the skilled person in the art should understand that the technical solutions of the present disclosure can be modified or replaced, which shall not deviate from the spirit and scope of the technical solutions of the present disclosure.

## Claims

1. A radio frequency ablation apparatus, comprising:
a radio frequency energy generation unit configured to generate and output radio frequency power required for radio frequency ablation;
an ablation component electrically connected to the radio frequency energy generation unit and configured to transmit the radio frequency power output by the radio frequency energy generation unit to an ablation site of heart, so as to perform radio frequency ablation on the ablation site;
a signal acquisition unit comprising at least an electrocardiogram (ECG) signal acquisition unit, the ECG signal acquisition unit comprising a plurality of electrodes configured to contact with a plurality of different body surface sites of a patient to acquire multi-lead ECG signal(s) represented by cardiac electrophysiological activity(s) acting on the body surface during radio frequency ablation; and
a control unit electrically connected to the ECG signal acquisition unit and the radio frequency energy generation unit, respectively, the control unit being configured to receive the multi-lead ECG signal(s) and control the radio frequency power output by the radio frequency energy generation unit according to the multi-lead ECG signal(s).

2. The radio frequency ablation apparatus according to claim 1, wherein when the control unit controls the radio frequency power output by the radio frequency energy generation unit according to the multi-lead ECG signal(s), it specifically analyzes a real-time heart rate according to the multi-lead ECG signal(s), and controls the radio frequency power output by the radio frequency energy generation unit according to the analysis result.

3. The radio frequency ablation apparatus according to claim 2, wherein the control unit is further configured to control the radio frequency energy generation unit to stop outputting the radio frequency power when the real-time heart rate does not fall within a heart rate threshold range;
the control unit is further configured to control the radio frequency energy generation unit to stop outputting the radio frequency power when a change rate of heart rate is greater than a first change rate threshold of heart rate, wherein the change rate of heart rate is a change rate of the real-time heart rate relative to an initial heart rate; and
the control unit is further configured to control the radio frequency energy generation unit to stop outputting the radio frequency power when an absolute value of a variation rate of heart rate is not less than a variation rate threshold of heart rate, wherein the variation rate of heart rate is a variation rate of the real-time heart rate relative to the initial heart rate.

4. The radio frequency ablation apparatus according to claim 3, wherein the radio frequency ablation apparatus further comprises a first counting module electrically connected to the control unit and configured to count the number of times that the change rate of heart rate is continuously greater than a second change rate threshold of heart rate, wherein the second change rate threshold of heart rate is less than the first change rate threshold of heart rate;
the control unit is further configured to control a count value of the first counting module to add one when the change rate of heart rate is greater than the second change rate threshold of heart rate, and determine whether the count value of the first counting module is greater than a first count threshold; and
the control unit is further configured to control the radio frequency energy generation unit to pause outputting the radio frequency power when the count value of the first counting module is greater than the first count threshold, and control the first counting module to be cleared to zero.

5. The radio frequency ablation apparatus according to claim 4, wherein the control unit is further configured to control the radio frequency energy generation unit to reduce the output radio frequency power when the count value of the first counting module is not greater than the first count threshold.

6. The radio frequency ablation apparatus according to claim 1 or 2, wherein when the control unit controls the radio frequency power output by the radio frequency energy generation unit according to the multi-lead ECG signal(s), it further specifically analyzes a real-time ST segment amplitude according to the multi-lead ECG signal(s), and control the radio frequency power output by the radio frequency energy generation unit according to the analysis result.

7. The radio frequency ablation apparatus according to claim 6, wherein the control unit is further configured to control the radio frequency energy generation unit to stop outputting the radio frequency power when an offset of ST segment amplitude is not less than an offset threshold of ST segment amplitude, wherein the offset of ST segment amplitude is an offset of the real-time ST segment amplitude relative to an initial ST segment amplitude; and
the control unit is further configured to control the radio frequency energy generation unit to stop outputting the radio frequency power when an absolute value of a variation rate of ST segment amplitude is not less than a variation rate threshold of ST segment amplitude, wherein the variation rate of ST segment amplitude is a variation rate of the real-time ST segment amplitude relative to the initial ST segment amplitude.

8. The radio frequency ablation apparatus according to claim 6, wherein the radio frequency ablation apparatus further comprises a second counting module electrically connected to the control unit and configured to count the number of times that a change rate of ST segment amplitude is continuously greater than a change rate threshold of ST segment amplitude, wherein the change rate of ST segment amplitude is a change rate of the real-time ST segment amplitude relative to the initial ST segment amplitude;
the control unit is further configured to control a count value of the second counting module to add one when the change rate of ST segment amplitude is greater than the change rate threshold of ST segment amplitude, and determine whether the count value of the second counting module is greater than a second count threshold; and
the control unit is further configured to control the radio frequency energy generation unit to pause outputting the radio frequency power when the count value of the second counting module is greater than the second count threshold, and control the second counting module to be cleared to zero.

9. The radio frequency ablation apparatus according to claim 8, wherein the control unit is further configured to control the radio frequency energy generation unit to reduce the output radio frequency power when the count value of the second counting module is not greater than the second count threshold.

10. The radio frequency ablation apparatus according to claim 1, wherein the radio frequency ablation apparatus further comprises a human-computer interaction unit electrically connected to the control unit and configured to receive ablation parameters set by an operator and transmit the set ablation parameters to the control unit; and
the control unit is further configured to control the radio frequency power output by the radio frequency energy generation unit according to the set ablation parameters, wherein the set ablation parameters comprise ablation time and initial radio frequency power.

11. The radio frequency ablation apparatus according to claim 1, wherein the signal acquisition unit further comprises a power acquisition unit electrically connected to the control unit and configured to acquire actual power signal(s) acting on the ablation site by the radio frequency energy generation unit, and transmit the actual power signal(s) to the control unit; and
the control unit is further configured to control the radio frequency power output by the radio frequency energy generation unit according to the actual power signal(s).

12. The radio frequency ablation apparatus according to claim 1, wherein the signal acquisition unit further comprises an impedance acquisition unit electrically connected to the control unit and configured to collect impedance signal(s) of the ablation site and transmit the impedance signal(s) to the control unit; and
the control unit is further configured to control the radio frequency power output by the radio frequency energy generation unit according to the impedance signal(s).

13. The radio frequency ablation apparatus according to claim 1, wherein the signal acquisition unit further comprises a temperature acquisition unit electrically connected to the control unit and configured to collect temperature signal(s) of the ablation site and transmit the temperature signal(s) to the control unit; and
the control unit is further configured to control the radio frequency power output by the radio frequency energy generation unit according to the temperature signal(s).
